# EUROPEAN PATENT SPECIFICATION

(11) **EP 4 431 134 B1**
(45) Date of publication and mention of the grant of the patent: **26.03.2025**
(21) Application number: 23170089.9
(22) Date of filing: 26.04.2023
(51) Int. Cl.: A61M 5/32, A61M 5/46

(54) **MEDICAL DEVICE FOR INTRADERMAL INJECTION, METHOD FOR INTRADERMAL INJECTION AND GUIDING DEVICE FOR USE IN SUCH A METHOD**
MEDIZINISCHE VORRICHTUNG ZUR INTRADERMALEN INJEKTION, VERFAHREN ZUR INTRADERMALEN INJEKTION UND FÜHRUNGSVORRICHTUNG ZUR VERWENDUNG IN SOLCH EINEM VERFAHREN
DISPOSITIF MÉDICAL POUR INJECTION INTRADERMIQUE, PROCÉDÉ D'INJECTION INTRADERMIQUE ET DISPOSITIF DE GUIDAGE DESTINÉ À ÊTRE UTILISÉ DANS UN TEL PROCÉDÉ

(30) Priority: 14.03.2023 IN 202321017135
(43) Date of publication of application: 18.09.2024
(73) Proprietor: Kairish Innotech Private Ltd., Mumbai 400021 (IN)
(72) Inventor: DADACHANJI, Rishad Kairus, 400005 Mumbai (IN); POTDAR, Pratul Prakash, 396210 Nani Daman (IN); PATEL, Keyurkumar Arvindbhai, 396210 Daman, Daman & Diu (IN); CHUDASMA, Krupal Ashokbhai, 382345 Ahmedabad (IN)
(74) Representative: 2K Patentanwälte Blasberg Kewitz & Reichel

(56) References cited:
- WO-A1-2014/137901
- WO-A1-2023/285151
- US-A1- 2010 137 831
- US-A1- 2016 367 766

## Description

### Field of Invention

The present invention generally relates to drug delivery devices for injection, and more specifically relates to medical device for intradermal injection of a dosage to a subject, to a method for intradermal injection of a dosage to a subject and to a guiding device (intradermal adapter) for use in such a method.

### Background of Invention

Effective drug delivery to the intradermal or shallow subcutaneous (hypodermis) space of a subject requires a precise positioning and guiding of an injection device, such as a syringe, on a skin surface, for precisely defining the penetration depth of an injection needle in the skin surface and the inclination angle of the injection needle during injection. Positioning and guiding an injection device become very challenging particularly if it comes to intradermal injection of a dosage to a subject under relatively small acute angles, such as 15 degrees, and very shallow penetration depths, e.g. of the order of about 2.5 mm, which are to be understood as typical conditions of practice of the device and method of the present application.

US 10,143,810 B2 discloses a needle guide with a base plate and a guiding platform whose angle relative to the base plate can be adjusted. The guiding platform includes a slot that receives a portion of either a needle or a catheter, and guides the needle or catheter for injection.

US 9,655,686 B2 discloses a pistol-shaped guide for guiding the movement of a syringe under a precisely defined angle for injection.

US 8,430,889 B2 discloses a puncture needle holder where the penetration angle of an injection device can be precisely defined.

US 8,083,715 B2 discloses a needle assembly for intradermal injection, comprising an adapter which can adjust the angle of injection, and a tubular guiding cylinder that receives and guides movement of a syringe.

US 10,413,681 B2 discloses an adapter with an injection needle for intradermal injection perpendicular to the skin surface of a subject. The adapter is placed on the skin surface of a subject and coupled with the distal end of an injection syringe.

US 2004/0147901 A1 discloses a device for intradermal injection by means of a syringe, including a tubular housing receiving the whole syringe. The tubular housing has a slanted front end with a base for placement on the skin surface, which defines the angle of penetration of the syringe needle. At the proximal end of the device, a special plunger assembly is provided that is configured to push the plunger rod downward, for injecting a dosage. Slots are provided for defining the penetration depth of the injection needle in the skin.

US 2016/0367766 A1 discloses a medical device according to the preamble of claim 1, for training staff to properly conduct an injection, which may be an intradermal injection. In certain embodiments, the device includes a tubular housing configured to receive and guide a syringe for injection under a predetermined angle relative to the skin surface of a subject. The front end of the tubular housing is broadened to a planar base surface for placement on the skin surface of a subject. The distal end of the syringe is relatively far away from the skin surface. Measures to control the penetration depth of the needle in the skin surface are not disclosed.

US 2010/137831 A1 discloses a guiding device for positioning and guiding an injection device for intradermal injection of a dosage to a subject. The contact surface of the base is not planar, but comprises a first and second primary skin contacting surface, which are provided on generally different, adjacent planes and positioned at an acute angle relative to each other, which is required to ensure a certain deformation of a target tissue site when the adapter is applied to the skin of the subject, so that in use the skin of the subject conforms to the first and second primary skin contacting surfaces of the adapter, and the needle cannula penetrates through the epidermis and dermis at a shallow angle to the surface of the skin. Moreover, a sleeve-shaped guiding portion that tightly matches the predetermined profile of the injection device at its distal end is not disclosed.

### Summary of Invention

It is an object of the present invention to provide an enhanced guiding device for positioning and guiding an injection device for intradermal injection of a dosage to a subject under repeatable and precisely defined conditions, having a simple and low-cost configuration and enabling a fool-proof handling even by inexperienced or not well-trained staff. Further aspects of the present invention relate to a medical device including such a guiding device and to a method for controlling intradermal injection of a dosage to a subject by means of an injection device.

These problems are solved by a guiding device for positioning and guiding an injection device for intradermal injection of a dosage to a subject as claimed by claim 1, by a medical device as claimed by claim 9, and by a method for controlling intradermal injection of a dosage to a subject by means of an injection device as claimed by claim 10. Further advantageous embodiments are the subject-matter of the dependent claims.

According to the present invention there is provided a guiding device for positioning and guiding an injection device for intradermal injection of a dosage to a subject, said injection device comprising a syringe body having a predetermined profile at a distal end thereof and a needle having a distal needle end adapted to penetrate a skin surface of the subject for intradermal injection of the dosage, said guiding device comprising a base having a contact surface for alignment with the skin surface of the subject and a tubular guiding device configured for guiding an axial movement of the injection device toward the skin surface from a retracted first state where the distal needle end does not penetrate the skin surface and is shielded against the skin surface by a portion of the guiding device to a second state where the distal needle end penetrates the skin surface of the subject, said tubular guiding device defining a central axis slanted relative to the contact surface at an acute angle and comprising a distal opening to be passed by the distal needle end for intradermal injection, wherein the tubular guiding device comprises at least one positive-fit locking member for locking the foremost distal position of the injection device in the tubular guiding device in a positive-fit manner and coupling the injection device to the guiding device in the second state of the injection device.

According to the present invention, the contact surface of the base is planar and the tubular guiding device is slanted relative to the base at said acute angle, wherein the tubular guiding device comprises a sleeve-shaped guiding portion that tightly matches the predetermined profile at the distal end of the injection device at least in sections, for preventing a tilting of the injection device about the central axis at or near the foremost distal position of the injection device in the tubular guiding device, and the at least one positive-fit locking member is provided at a side proximal to the sleeve-shaped guiding portion.

A further aspect of the present invention, as claimed by claim 9, relates to a medical device for intradermal injection of a dosage to a subject including such a guiding device.

A further aspect of the present invention, as claimed by claim 10, relates to a method for controlling intradermal injection of a dosage to a subject by means of an injection device, in particular for controlling the angle and depth of penetration of the needle in the skin surface of a subject for injection.

### Technical Effects

As the sleeve-shaped guiding portion of the tubular guiding device tightly matches the profile at the distal end of the injection device, any tilting of the injection device at and near the foremost distal position in the tubular guiding device can be reliably prevented. Thus, the conditions of the injection device at the stage, when the needle pierces and finally penetrates the skin surface for injection, can be precisely defined. The needle will be guided straight in axial direction and thus pierces and penetrates the skin surface precisely under the acute angle defined by the tubular guiding device without significant deviations, such as tilting.

The sleeve-shaped guiding portion of the tubular guiding device may also serve as a stopping member to delimit the axial movement of the injection device in the tubular guiding device and thus precisely define its foremost distal position and the penetration depth of the needle in the skin surface.

The positive-fit locking member provided at a side proximal to the sleeve-shaped guiding portion reliably locks the foremost distal position of the injection device in the tubular guiding device and couples the injection device to the guiding device. Locking of the injection device by means of the positive-fit locking member may generate a clearly audible noise or sound, e.g. caused by the snapping-back of resilient locking fingers to their relaxed home position, that indicates that the medical device is ready for injection.

Moreover, the injection device can only be removed from the skin surface after injection together with the guiding device, as the injection device is coupled to the tubular guiding device by the positive-fit locking member and remains coupled to the tubular guiding device also after the injection is completed. Thus, also during removal of the needle from the skin surface the angle and direction of pulling out the needle from the tissue at the injection site can be precisely defined by the guiding device. Hence, also removal of the needle is very smooth, preventing unnecessary destruction of tissue at the injection site.

By means of a guiding device according to the present invention, medical injection becomes fool-proof and can be executed even by inexperienced and untrained personnel, because both the inclination angle and penetration depth of the needle in the skin surface of a subject is precisely defined by the guiding device at all stages of injection and because the guiding device leaves no possibility of operating the medical device during injection but in a standard and clearly prescribed manner.

### Overview on Drawings

The invention will now be described by way of example and with reference to the accompanying drawings, from which further features, advantages and problems to be solved will become apparent. In the drawings:
- Figs. 1a to 1e: show a first embodiment of a guiding device for use with an injection device according to the present invention;
- Fig. 2a: shows an example of an injection device for use with a guiding device according to the present invention, embodied as a syringe;
- Fig. 2b: shows detail A of Fig. 2a on an enlarged scale;
- Figs. 3a to 3d: show the insertion of a syringe into and guiding of a syringe by the guiding device of the first embodiment according to the present invention at four different stages; and
- Fig. 3e: shows the locking of the syringe in the guiding device of the first embodiment according to the present invention in a foremost distal position of the syringe, for intradermal injection of a dosage to a subject.

In the drawings, the same reference numerals designate identical or substantially equivalent elements or groups of elements.

### Detailed description of preferred embodiments

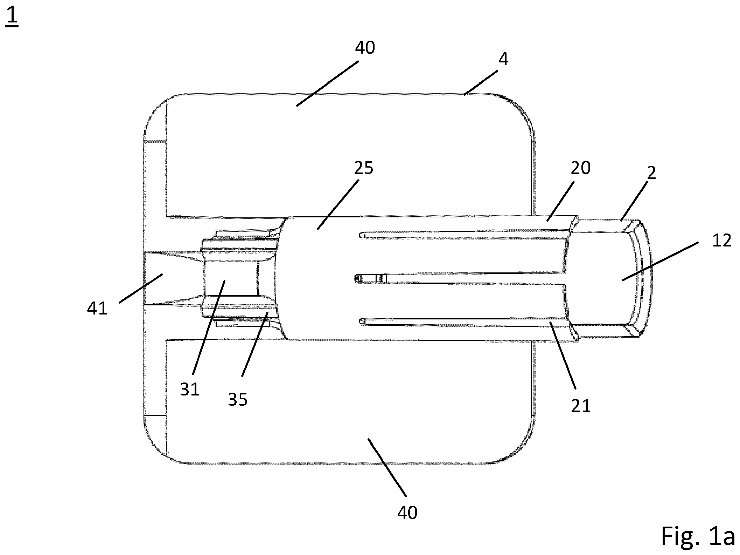

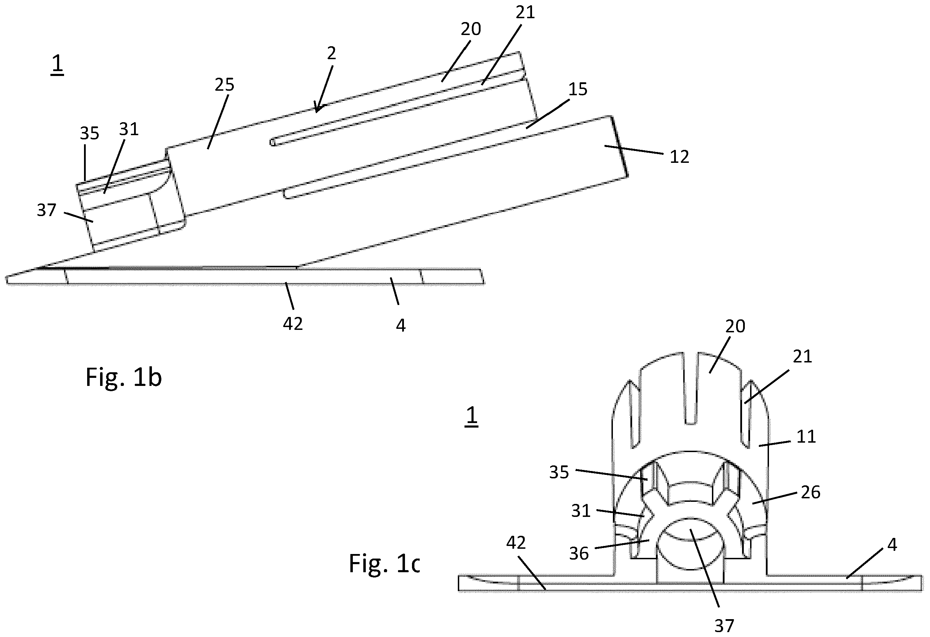

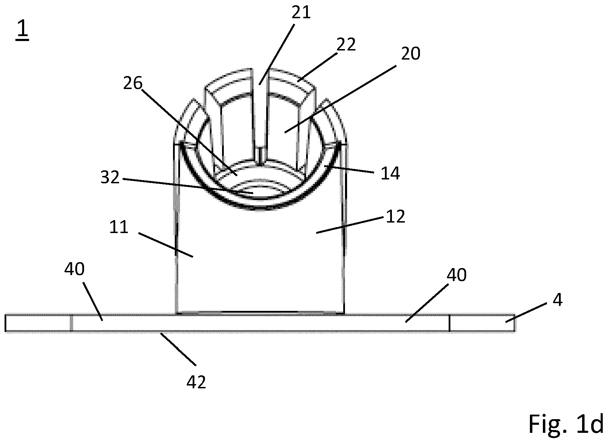

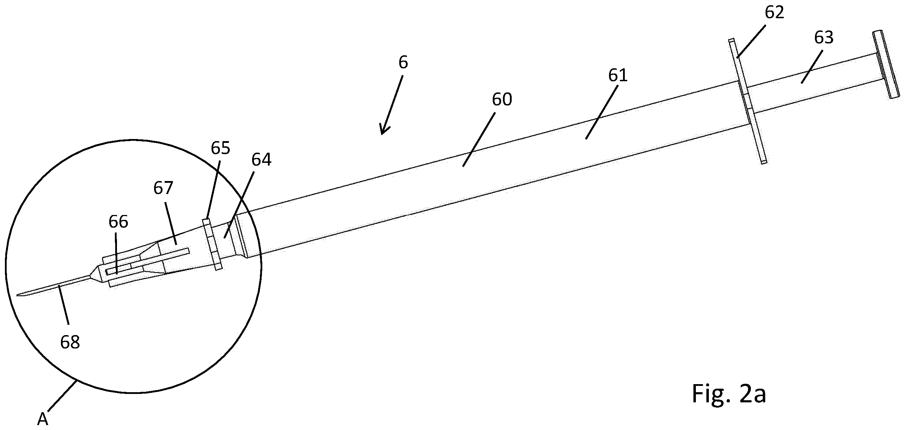

Figs. 1a to 1e show a guiding device 1 according to the present invention, intended for positioning and guiding an injection device, such as a syringe shown in Fig. 2a, for intradermal injection of a dosage to a subject. The guiding device is integrally made of a plastic material, e.g. by injection molding, and generally comprises a tubular guiding device 2 and a basis 4, which is preferably integrally formed with the tubular guiding device 2, but may also be connected with the tubular guiding device 2. The tubular guiding device 2 defines a central axis L that is slanted relative to the basis 4 under an acute angle α, which corresponds to the angle under which the needle of an injection device such as a syringe is to penetrate the skin surface of a subject for injection. This angle α may be of the order of 15 degrees, but may be any angle in a wider range e.g. between 5 degrees and 25 degrees, more preferably in a range between 10 degrees and 20 degrees, and even more particularly in a range between 12.5 degrees and 17.5 degrees, depending on the specific application. The base 4 is generally planar and comprises a planar contact surface 42 at a bottom thereof, which is positioned on the skin surface of a subject for injection, e.g. on the inner forearm of a subject, for alignment with the skin surface. For this purpose, the base 4 may comprise two lateral portions 40 each protruding laterally from the tubular guiding device 2 and forming finger support areas supporting the fingers of an operating person when pushing the base 4 with his/her fingers onto the skin surface of the subject for injection. These lateral portions 40 may be slightly bent or bulged, for better conformance e.g. with the inner forearm of a subject.

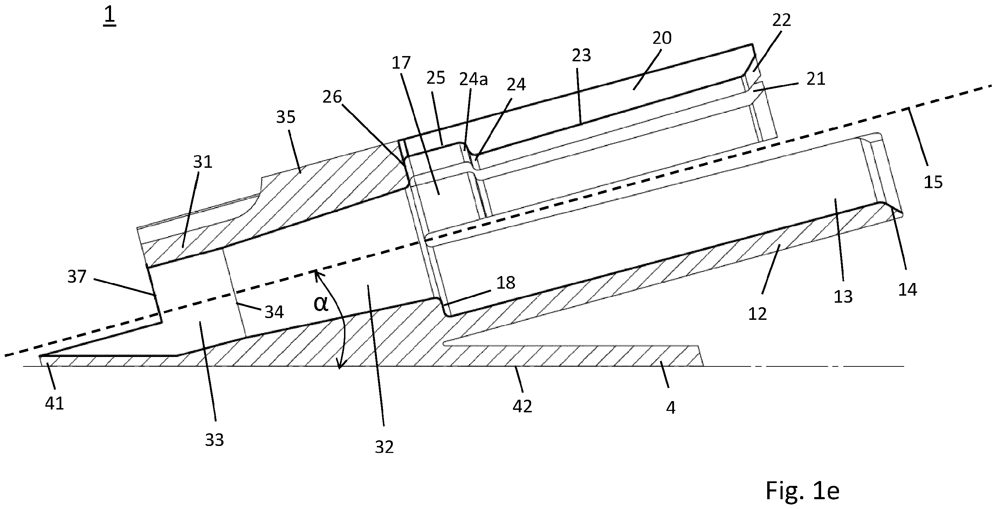

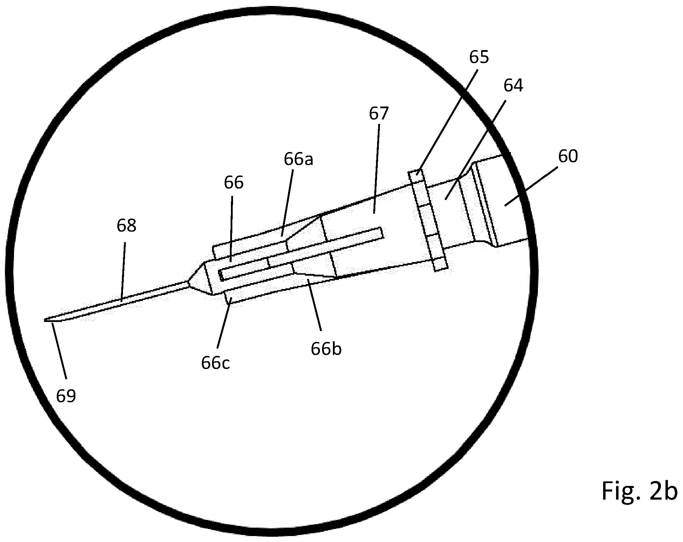

The inner profile of the tubular guiding device 2 generally corresponds to the outer profile of the injection device, and is usually circular, although other profiles, such as square-shaped profiles, may also be envisaged. The tubular guiding device 2 includes, at a proximal end thereof, a proximal tubular guiding device 11, which is generally a little wider than the injection device to be guided, to ease insertion of the injection device. For this purpose, the profile of the proximal tubular guiding device 11 may be tapered towards the distal end of the guiding device 1. More specifically, the proximal tubular guiding device 11 in the shown embodiment includes a bottom side-wall 12, which is semi-circular in profile and extends in parallel with the central axis L and a plurality of resilient locking fingers 20, which are separated by two longitudinal central slots 15 from the bottom side-wall 12. The bottom side-wall 12 may be a little longer than the locking fingers 20, to ease catching the injection device in the proximal tubular guiding device 2. The plurality of resilient locking fingers 20 are disposed preferably at equiangular intervals around the central axis L and opposite to the bottom side-wall 12, and the resilient locking fingers 20 are separated by longitudinal slots 21 from each other. The stems 25 of the resilient locking fingers 20 are interconnected to form a circular structure together with the distal end of the bottom side-wall 12 at a side distal to the radial steps 18, 26. Each resilient locking finger 20 comprises a tapered guiding surface 23, which is each tapered toward the distal end of the proximal tubular guiding device 2 and each terminates in a locking nose 24 protruding from a respective stem 25 of a resilient locking finger 20. The stem 25 together with a flank of the locking nose 24 and an opposite radial step 26 forms a receptacle, which is generally of rectangular shape, if viewed in the cross-sectional view of Fig. 1e, and suited to accommodate a protrusion provided at a distal end of the injection device, such as the protrusion 65 at the distal end of the syringe 6 shown in Figs. 2a and 2b. The flank 24a of the locking nose 24 may be slanted under a relatively small acute angle relative to a vertical onto the central axis L, whereas the radial step 26 preferably extends perpendicular to the central axis L. The bottom side-wall 12 of the proximal tubular guiding device 11 is relatively stiff and serves as a reference surface for precisely guiding an axial movement of the injection device in the proximal tubular guiding device 11, whereas the resilient locking fingers 20 are less stiff than the bottom side-wall 12 and may be spread apart resiliently during insertion of the injection device into the proximal tubular guiding device 11 to thereby push the injection device against the bottom side-wall 12, as outlined below in more detail.

The proximal tubular guiding device 11 is followed by a distal tubular guiding device 30, which generally is less wide and generally serves for preventing a tilting of the injection device about the central axis L at or near the foremost distal position of the injection device in the tubular guiding device 30. For this purpose, the tubular guiding device 30 comprises at least one sleeve-shaped guiding portion, of which a first portion directly following the proximal tubular guiding device 11 may be formed as a tapered guiding sleeve 32, as shown in Fig. 1e. Moreover, a cylindrical guiding sleeve 33 may also be provided at a side distal to the tapered guiding sleeve 32.

As will become apparent from a comparison of Fig. 1e with the specific profile at the distal end of the injection device 6 shown in Figs. 2a and 2b, the specific shape of the at least one guiding sleeve 32, 33 at the distal end of the tubular guiding device depends on the specific profile at the distal end of the injection device 6, and the main characteristic of the at least one guiding sleeve 32, 33 is that its profile tightly matches the profile at the distal end of the injection device at least in sections and over a distance in axial direction of the injection that is sufficient to reliably prevent a tilting of the injection device about the central axis L once the distal end of the injection device is accommodated in the at least one guiding sleeve 32, 33 for injection, which corresponds to the position at or near the foremost distal position of the injection device in the tubular guiding device 30. To simplify the present disclosure, in the following it is assumed that the profile at the distal end of the injection device is tapered, as shown in Figs. 2a and 2b. The present invention shall, however, not be construed to be delimited to such a specific profile at the distal end of the injection device.

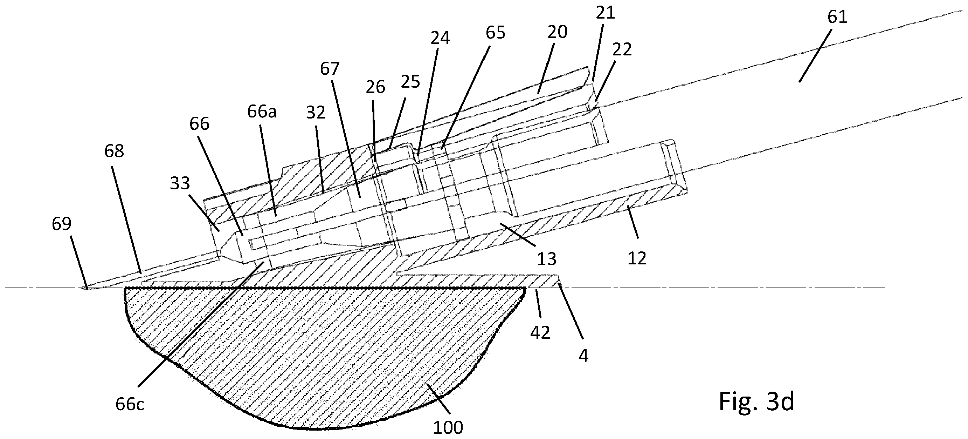

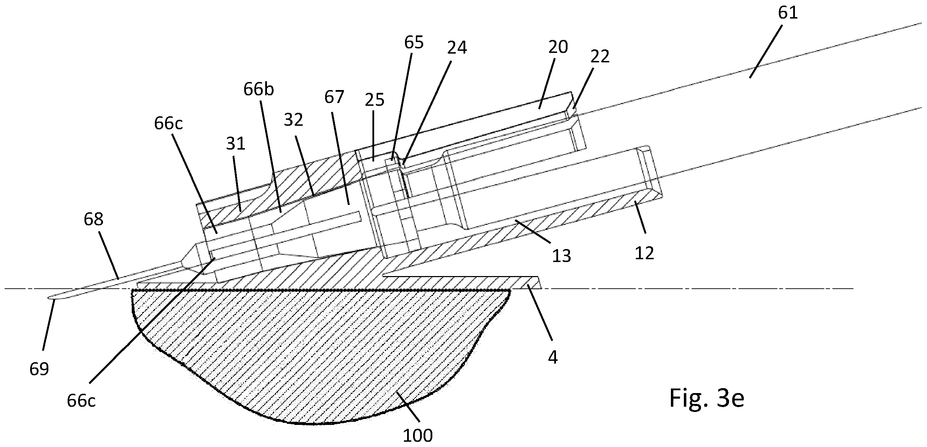

The circular side-wall 31 of the cylindrical guiding sleeve 33 forms a distal opening 37, through which the needle 68 of the injection device passes at or near its foremost axial position in the tubular guiding device 2 for injection, as shown in Figs. 3d and 3e. The circular side-wall 31 of the guiding sleeves 32, 33 is stiffened by axial stiffening ribs 35 and by an arc-shaped structure 36 formed on the outer surface of the side-wall 31, to substantially prevent any widening of the guiding sleeves 32, 33 upon insertion of the distal end of the injection device, so that the guiding sleeves 32, 33 basically define constant inner profiles during use.

In the following, the general configuration of an injection device 6 for use with the above guiding device 1 will be described with reference to the preferred example of a syringe shown in Figs. 2a and 2b. The syringe 6 comprises a cylindrical syringe body 60 of constant outer diameter, which defines a storing chamber for storing a liquid drug or solution to be administered by injection. At the proximal end of the syringe body 61 a finger rest 62 is provided, which enables to control use of the syringe 6 with the forefinger and middle finger and pushing down the plunger rod 63 with the thumb. In this example, a tapered profile is provided at the distal end 64 of syringe body 60. More specifically, a tubular needle mount 66 is mounted to the distal end 64 of syringe body 60, which is formed as a seat fixedly holding the needle 68 and establishing a fluid communication between the needle 68 and the storage chamber 61. The distal end 69 (needle tip) of needle 68 is beveled. The tubular needle mount 66 comprises a conical, tapered portion 67, and at the distal end of this tapered portions 67 a plurality of ribs 66a extend in axial direction, which together define a straight, cylindrical outer profile at the foremost distal end of needle mount 66. At the proximal end of needle mount 66 a circular, radial protrusion 65 is provided, which extends outward and perpendicular to the central axis of syringe 6. The distance between this radial protrusion 65 and the needle end 69 is precisely defined and represents an important characteristic for precisely defining the penetration depth of needle 68 in the skin surface of a subject during injection. Moreover, the outer profile of the needle mount 66, or more generally of the distal end of the syringe 6 represents an important characteristic for preventing a tilting of the syringe (injection device) about the central axis L in the foremost distal position of the syringe in the tubular guiding device 2.

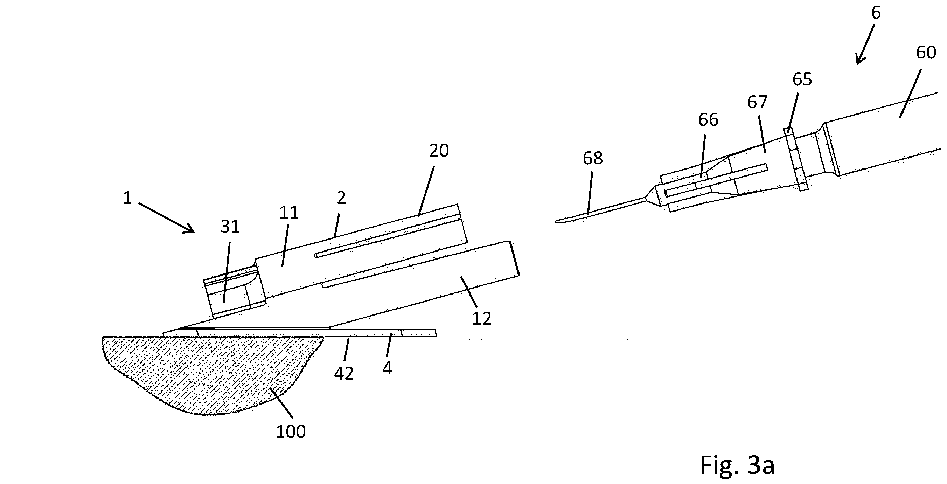

In the following, the insertion of a syringe 6 into and guiding of a syringe by the guiding device 1 will be described in more detail with reference to Figs. 3a to 3e. At a first stage, as shown in Fig. 3a, the guiding device 1 is positioned on the skin surface 100 of a subject, by placing the contact surface 42 of base 4 on the skin surface 100 at a desired location, such as the inner forearm of a subject. For a proper alignment of the base 4 with the skin surface 100, the guiding device 1 may be pushed perpendicular to the skin surface 100 during the entire injection process, e.g. by pushing the two lateral portions 40 of base 4 onto the skin surface 100 with two fingers, such as the thumb and fore-finger. Once the guiding device 1 is properly positioned on the skin surface, the syringe 6 is moved towards the guiding device 1, generally along the central axis of the tubular guiding device 2, until the needle 68 enters the proximal tubular guiding device 11. In order to ease catching the radial protrusion 65 of syringe 6, the proximal end surface of the bottom side-wall 12 may be provided with an insertion bevel 14 and the proximal end surfaces of locking fingers 20 may be provided with insertion bevels 22. Moreover, the bottom side-wall 12 may be a little longer than the locking fingers 20, as shown in Fig. 3a. At this stage, which represents a retracted first stage of syringe 6, the beveled needle end 69 is preferably shielded against the skin surface 100 by a portion of guiding device 1, in particular by the bottom of the cylindrical guiding sleeve 33 and the distal end 41 of base 4.

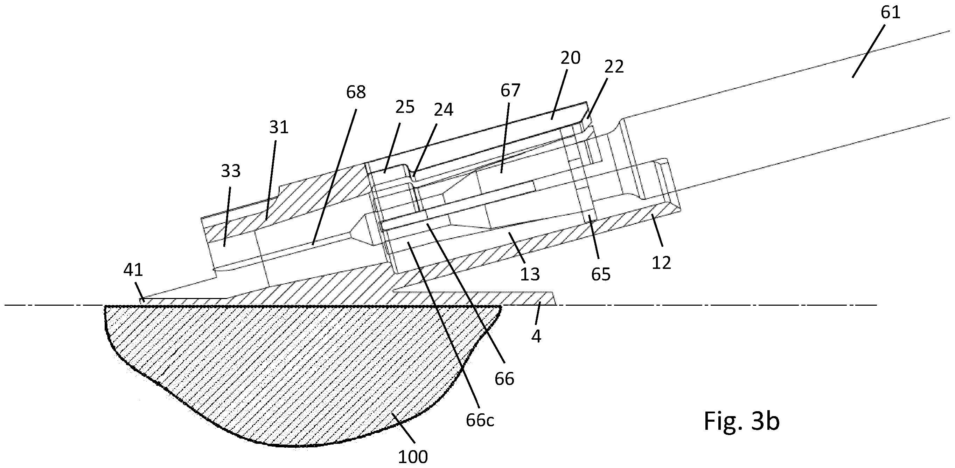

As shown in Fig. 3b, the radial protrusion 65 of syringe 6 (or more generally the outer surface of a portion of syringe 6) continues sliding along the tapered guiding surfaces 23 of the locking fingers 20 and the bottom side-wall 12, until the cylindrical portion 66c of needle mount 66 finally enters the cylindrical guiding sleeve 33, so that the syringe 6 starts to be aligned more or less perfectly with the central axis L of tubular guiding device 2. At this stage, which still represents a retracted first stage of syringe 6, the beveled needle end 69 preferably remains shielded against the skin surface 100 by a portion of guiding device 1, in particular by the bottom of the cylindrical guiding sleeve 33 and the distal end 41 of base 4. At this stage, the resilient locking fingers 20 start to be spread apart due to cooperation with the radial protrusion 65 of syringe 6, and the syringe 6 is pushed perpendicular to central axis L towards the bottom side-wall 12 by the resilient locking fingers 20. The bottom side-wall 12 thus serves as a reference surface at all stages of injection. Moreover, the syringe 6 may still tilt by a relatively small angle only about the central axis of tubular guiding device 2, because the gap between the side-wall 31 of tapered guiding sleeve 32 and the cylindrical portion 66c of needle mount 66 is relatively narrow already.

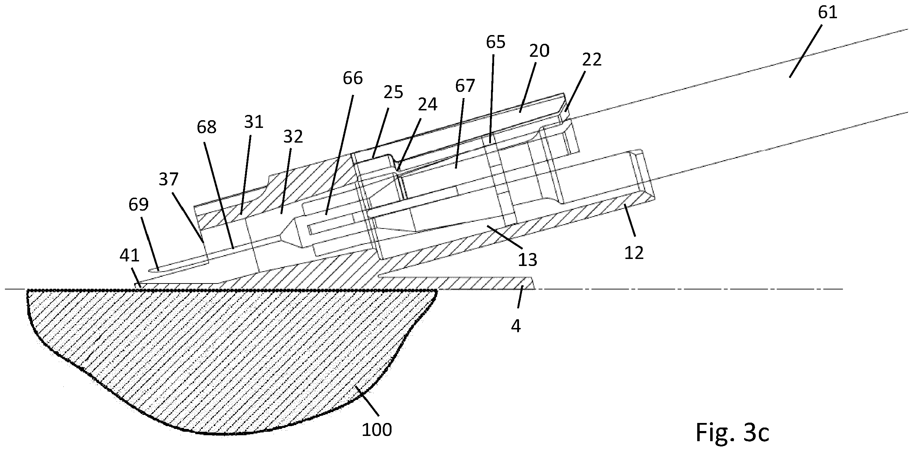

As shown in Fig. 3c, finally the beveled needle end 69 leaves the cylindrical guiding sleeve 33 and passes the distal opening 37. The radial protrusion 65 of syringe 6 (or more generally the outer surface of a portion of syringe 6) continues sliding along the tapered guiding surfaces 23 of the locking fingers 20 and the bottom side-wall 12, and the gap between the side-wall 31 of tapered guiding sleeve 32 and the cylindrical portion 66c of needle mount 66 gradually becomes narrower. As the syringe 6 is further moved in axial direction, the resilient locking fingers 20 are increasingly spread apart, until the stage shown in Fig. 3d is finally reached.

At this stage, the resilient locking fingers 20 are spread apart significantly, pushing the radial protrusion 65 and thus the proximal portion of syringe 6 towards the bottom side-wall 12 of the proximal tubular guiding device 11. At this stage, the cylindrical portion 66c of needle mount 66 is already accommodated in the cylindrical guiding sleeve 33, the profile of which tightly matches the outer profile of cylindrical portion 66c of needle mount 66 is. Hence, at this stage, where the beveled needle end 69 starts piercing the skin surface, the syringe is already perfectly centered in tubular guiding device 2 and aligned with central axis L. At this stage, the radial protrusion 65 has nearly reached the locking noses 24.

When the syringe 6 is moved further in axial direction, the beveled needle end 69 finally penetrates the skin surface under the acute angle α defined by the tubular guiding device 2 and the radial protrusion 65 continues spreading the resilient locking fingers 20 further apart. Axial movement of syringe 6 is precisely guided along central axis L, as the cylindrical portion 66c of needle mount 66 is tightly accommodated in cylindrical guiding sleeve 33, without radial play. Moreover, the radial protrusion 65 and thus the proximal portion of syringe 6 is pushed towards the bottom side-wall 12 of the proximal tubular guiding device 11 by the resilient locking fingers 20. Hence, any tilting of syringe 6 about central axis L is reliably prevented.

Finally, the radial protrusion 65 starts sliding over the locking noses 24 and along the flanks 24a of locking noses 24. At this stage, the resilient locking fingers 20 snap back to their relaxed home position shown in Fig. 3e. This snapping back of the resilient locking fingers 20 results in a characteristic audible clicking-noise or sound that will be clearly perceived by the person operating syringe 6 and guiding device 1, and represents a clear and unambiguous signal to that person indicating that the syringe 6 has reached its foremost distal position and is ready for injection of the dosage to the subject by pushing the plunger rod 63 into syringe body 60.

At this stage, the tapered portion 67 of needle mount 66 completely abuts against the tapered inner surface of tapered guiding sleeve 32. As the flanks 24a of locking noses 24 may be slightly tilted relative to a vertical onto central axis L, the radial protrusion 65 and hence the distal end of syringe 6 may be resiliently biased towards the tapered inner surface of tapered guiding sleeve 32. This foremost distal position of syringe 6 in tubular guiding device precisely defines the penetration depth of needle 68 in the skin surface 100. At this stage, the beveled needle end 69 projects beyond the bottom contact surface 42 of base 4. As an alternative, a semi-circular aperture may be formed at the front portion of base 4, in the are designated by reference numeral 41 in Fig. 1a, and the needle end 69 may pass this aperture for piercing and finally penetrating the skin of the subject.

In order to control the angular orientation of the beveled needle end 69, an indexing feature may be provided at the distal end of the tubular guiding device 2, in particular at the distal end of the tapered and/or cylindrical guiding sleeve 32, 33, such as a protrusion or rib, which cooperates with a corresponding feature on the outer surface of the syringe 6, in particular at the outer surface of the tubular needle mount 66. Such an indexing feature may be formed in particular as a guiding rib formed on the outer surface of the tubular needle mount 66, for determining the orientation of the beveled needle end 69 by cooperation with the corresponding indexing feature of the tubular guiding device 2.

The medical device consisting of guiding device 1 and syringe 6 can be operated easily by a person, preferably by placing and positioning the guiding device 1 on a skin surface 100 with one hand and then inserting syringe 6 into the tubular guiding device 2 and operating syringe 6 with the other hand.

By means of the guiding device 1 medical injection becomes fool-proof and can be executed even by inexperienced and untrained personnel, because both the inclination angle and penetration depth of needle 68 in the skin surface 100 is precisely defined by the guiding device 1 and because the guiding device 1 leaves no possibility of operating the medical device during injection in a different manner as outlined above.

As will become apparent to the skilled person when studying the above description, in the embodiment described above the tapered guiding sleeve 32 serves as a stopping member defining the foremost distal position of the syringe 6 in the tubular guiding device 2, namely by abutment of tapered portion 67 of needle mount 66 against the inner surface of tapered guiding sleeve 32. Once the distal end of the injection device 6 is accommodated in the tapered guiding sleeve 32, a further axial movement of the injection device is prevented. Thus, the penetration depth of needle 68 in the skin surface 100 is precisely defined.

As the injection device 6 is locked to the guiding device 1 in this foremost axial position by the at least one positive-fit locking member, namely in the afore-mentioned example by means of the locking noses 24, the injection device 6 can be removed from the skin surface 100 after injection of the dosage to the subject only together with the guiding device 1. For this purpose, the guiding device 1 together with the injection device 6 may be pulled a little backward from the injection site and then pulled away from the skin surface 100 along the axial direction defined by the central axis L, to ensure a smooth removal of the needle 68 without destroying tissue at the injection site.

After complete removal of the guiding device 1 from the skin surface 100, the injection device 6 can then be separated from the guiding device 1 by pulling the injection device 6 out of the tubular guiding device 2, thereby either overcoming the holding force exerted by the at last one positive-fit locking member or separating the distal end of the injection device 6, such as the tubular needle mount 66 disclosed above, from the injection device 6 and leaving the distal end together with the needle 68 locked to the tubular guiding device 2, for disposal.

According to the present invention, tolerances in the outer dimensions at the distal end of syringe 6 can be compensated, because the locking noses 24 of the resilient locking fingers 20 resiliently bias radial protrusion 65 and hence the distal end of syringe 6 against the tapered guiding sleeve 32, wherein the tapered guiding sleeve 32 serves as a stopping member for defining the foremost axial position of syringe 6. The, the penetration depth of the needle 68 in the skin surface 100 may be defined even more precisely. At the same time, the tapered guiding sleeve 32 tightly matches the predetermined profile at the distal end of the injection device 6 at least in sections, so that a tilting of the injection device 6 about the central axis L at or near the foremost distal position of the injection device 6 in the tubular guiding device 2 is reliably prevented. For this purpose, the tapered guiding sleeve 32 extends in axial direction over a certain distance allowing to precisely define and maintain the position of the distal end of the injection device 6 in radial direction. Preferably, the tapered guiding sleeve 32 and the cylindrical guiding sleeve 33 completely surround the distal end of syringe 6 at the foremost, locked position. It may be sufficient, however, if the tapered guiding sleeve 32 and the cylindrical guiding sleeve 33 about against the distal end of syringe 6 in sections, e.g. by means of axial ribs defining a contour tightly matching the predetermined profile at the distal end of syringe 6, e.g. of the tubular needle mount 66.

Of course, there exist many other options for implementing a stopping member preventing a further axial movement of the injection device (syringe) in the tubular guiding device beyond the stopping member. As an example, a stopping member may be formed as a protrusion provided in the sleeve-shaped guiding portion 32, 33 or elsewhere inside the tubular guiding device and cooperate with a corresponding contour, such as a radial protrusion or depression formed at a corresponding position on the outer surface of the injection device (syringe) 6, in a positive-fit manner. Generally, a stopping member may be implemented by means of frictional engagement and/or positive-fit. Frictional engagement may be between an outer contour at the distal end of the injection device (syringe) 6 and a corresponding profile formed inside the tubular guiding device 2, preferably at a foremost distal end thereof, such as the tapered guiding sleeve 32 or the cylindrical guiding sleeve 33. A proper frictional pairing between the material at the distal end of injection device (syringe) 6 and the material of the corresponding section of the tubular guiding device 2 may assist in establishing such a frictional engagement to define the foremost distal position of injection device (syringe) 6 inside the tubular guiding device 2.

While in the preferred embodiment outlined above, the radial protrusion 65 is part of a tubular needle mount 66 that is a member separate to the injection device (syringe) 6, it will become apparent to the skilled person when studying the above description, that such a radial protrusion, or also a depression, may be also be formed as an integral part of the injection device (syringe) 6. Such a radial protrusion or depression may also be part of a separate member, such as a sleeve, to be mounted near the distal end of the injection device (syringe) 6 at a predetermined location by imposing such a separate member on the distal end of the injection device (syringe) 6 or by clamping it at the injection device (syringe) 6 at a proper position.

As will become apparent to the skilled person when studying the above description, a medical device for intradermal injection of a dosage to a subject consists of an injection device and a guiding device as outlined above, which may be delivered to customers as a set consisting of two separate members or as individual members. Preferably, the injection device is a syringe. However, the present invention shall not be construed to be delimited to use of syringes only.

As will become apparent to the skilled person when studying the above description, a further aspect of the present invention relates to a guiding device (intradermal adapter) as outlined above, for use together with an injection device having a corresponding profile at least at a distal end thereof.

As will become apparent to the skilled person when studying the above description, a further aspect of the present invention relates to the use of a guiding device (intradermal adapter) as outlined above, together with an injection device having a corresponding profile at least at a distal end thereof, for intradermal injection of a dosage to a subject.

As will become apparent to the skilled person when studying the above description, a further aspect of the present invention relates to a method for intradermal injection of a dosage to a subject by means of an injection device, such as a syringe, and a corresponding guiding device (intradermal adapter), as outlined above.

## Claims

1. A guiding device (1) for positioning and guiding an injection device (6) for intradermal injection of a dosage to a subject, said injection device (6) comprising a syringe body (60) having a predetermined profile at a distal end thereof and a needle (68) having a distal needle end (69) adapted to penetrate a skin surface (100) of the subject for intradermal injection of the dosage;
said guiding device (1) comprising:
a base (4) having a contact surface (42) for alignment with the skin surface (100) of the subject, and
a tubular guiding device (2) configured for guiding an axial movement of the injection device (6) toward the skin surface (100) from a retracted first state where the distal needle end (69) does not penetrate the skin surface (100) and is shielded against the skin surface by a portion (12) of the guiding device (1) to a second state where the distal needle end (69) penetrates the skin surface (100) of the subject, said tubular guiding device (2) defining a central axis (L) slanted relative to the contact surface (42) at an acute angle (α) and comprising a distal opening (37) to be passed by the distal needle end (69) for intradermal injection, wherein
the contact surface (42) of the base (4) is planar and the tubular guiding device (2) is slanted relative to the base (4) at said acute angle (α), wherein
the tubular guiding device (2) comprises a sleeve-shaped guiding portion (32, 33) that tightly matches the predetermined profile at the distal end of the injection device (6), for preventing a tilting of the injection device (6) about the central axis (L) at or near the foremost distal position of the injection device (6) in the tubular guiding device (2), and
the tubular guiding device (2) comprises at least one locking member (24) provided at a side proximal to the sleeve-shaped guiding portion (32, 33) and configured for locking the injection device (6) to the guiding device (1) in the foremost distal position of the injection device (6) in the tubular guiding device (2) and coupling the injection device (6) to the guiding device (1) in the second state of the injection device (6),
**characterized in that**
the at least one locking member (24) is a positive-fit locking member configured for locking the injection device (6) to the guiding device (1) in the foremost distal position of the injection device (6) in the tubular guiding device (2) in a positive-fit manner and coupling the injection device (6) to the guiding device (1) in the second state of the injection device (6) such that the injection device (6) can be removed from the skin surface (100) after injection of the dosage to the subject together with the guiding device (1) while being coupled to the tubular guiding device (2).

2. The guiding device (1) as claimed in claim 1, wherein the at least one positive-fit locking member (24) is configured to resiliently bias the injection device (6) toward the foremost distal position of the injection device (6) in the tubular guiding device (2).

3. The guiding device (1) as claimed in claim 1 or 2, wherein
the sleeve-shaped guiding portion (32, 33) comprises a tapered guiding sleeve (32) near a distal end of the tubular guiding device (2) as a stopping member to limit the axial movement and define the foremost distal position of the injection device (6) in the tubular guiding device (2) by at least one of frictional engagement and positive-fit; or
the sleeve-shaped guiding portion (32, 33) or the tubular guiding device (2) comprises stopping member to limit the axial movement and define the foremost distal position of the injection device (6) in the tubular guiding device (2) by cooperation with the injection device (6), said stopping member comprising at least one protrusion provided in the sleeve-shaped guiding portion (32, 33).

4. The guiding device (1) as claimed in any of the preceding claims, wherein the tubular guiding device (2) further comprises a proximal tubular guiding device (11) disposed at a side proximal to the sleeve-shaped guiding portion (32, 33) and comprising at least one resilient locking finger (20), wherein
each positive-fit locking member (24) is provided at a respective resilient locking finger (20), and
each positive-fit locking member preferably is formed as a locking nose (24) or locking depression integrally formed with the respective resilient locking finger (20) and configured to cooperate with a corresponding protrusion (65) or depression provided at the injection device (6).

5. The guiding device (1) as claimed in claim 4, wherein the proximal tubular guiding device (11) is formed by a bottom side-wall (12) extending in parallel with the central axis (L) and a plurality of resilient locking fingers (20) disposed at equiangular intervals around the central axis (L) and opposite to the bottom side-wall (12), each resilient locking finger (20) comprising a tapered guiding surface (23), which is tapered toward the respective positive-fit locking member (24).

6. The guiding device (1) as claimed in claim 4 or 5, wherein each resilient locking finger (20) comprises a tapered guiding surface (23) tapered toward the distal end of the proximal tubular guiding device (2) and terminating in the locking nose (24) protruding from a respective stem (25) of the respective resilient locking finger (20).

7. The guiding device (1) as claimed in claim 6, wherein the stem (25) of the respective resilient locking finger (20) together with a flank of the respective locking nose (24) and a radial step (26) opposite along a direction of the central axis (L) forms a receptacle, which is generally of rectangular shape, if viewed in a cross-sectional view.

8. The guiding device (1) as claimed in any of the preceding claims, wherein the tubular guiding device (2) further comprises a cylindrical guiding sleeve (33) provided at a distal end thereof, configured to cooperate with a distal end of the injection device (6) at or near the foremost distal position of the injection device (6) in the tubular guiding device (2), for centering the injection device (6) in the tubular guiding device (2) when the distal needle end (69) penetrates the skin surface (100) of the subject for intradermal injection.

9. The guiding device (1) as claimed in any of the preceding claims, wherein the injection device (6) further comprises a tubular needle mount (66) mounted to the distal end of the syringe body (60) and the tubular needle mount (66) is formed as a seat fixedly holding the needle (68) and establishing a fluid communication between the needle (68) and a storing chamber (61) of the syringe body (60).

10. The guiding device (1) as claimed in claim 9, wherein the tubular needle mount (66) comprises a tapered portion (67) and a portion (66c) having a cylindrical outer profile disposed at a side distal to the tapered portion (67).

11. A medical device for intradermal injection of a dosage to a subject, comprising an injection device (6) comprising a syringe body (60) having a predetermined profile at a distal end thereof and a needle (68) having a distal needle end (69) adapted to penetrate a skin surface (100) of the subject for intradermal injection of the dosage, and
a guiding device (1) as claimed in any of the preceding claims, for guiding the axial movement of the injection device (6) toward the skin surface (100) from a retracted first state where the distal needle end (69) does not penetrate the skin surface (100) and is shielded against the skin surface by a portion (12) of the guiding device (1) to a second state where the distal needle end (69) penetrates the skin surface (100) of the subject.

12. A method for controlling an angle and depth of penetration of a needle in a skin surface of a subject by means of an injection device (6) comprising a syringe body (60) having a predetermined profile at a distal end thereof and a needle (68) having a distal needle end (69), comprising the steps of:
providing a guiding device (1) as claimed in any of claims 1 to 10;
placing the planar contact surface (42) of the base (4) on the skin surface (100) of the subject, for alignment of the base (4) with the skin surface (100) of the subject; and
introducing an injection device (6) into the tubular guiding device (2) and pushing the injection device (6) toward the skin surface (100) along a central axis (L) of the tubular guiding device (2) from a retracted first state where the distal needle end (69) does not penetrate the skin surface (100) and is shielded against the skin surface by a portion (12) of the guiding device (1) to a second state where the distal needle end (69) penetrates the skin surface (100) of the subject and the at least one positive-fit locking member (24) locks the injection device (6) to the tubular guiding device (1) in a positive-fit manner in the foremost distal position of the injection device (6) in the tubular guiding device (2) and couples the injection device (6) to the guiding device (1) in the second state of the injection device (6) to define a penetration depth of the needle (68) in the skin surface (100) of the subject, for controlling the angle and depth of penetration of the needle in the skin surface of the subject for injection and for preventing a tilting of the injection device (6) about the central axis (L) at or near the foremost distal position of the injection device (6) in the tubular guiding device (2) by cooperation of a sleeve-shaped guiding portion (32, 33) of the tubular guiding device (2) with the predetermined profile at the distal end of the injection device (6), wherein
the at least one positive-fit locking member (24) locks the injection device (6) to the guiding device (1) in the foremost distal position of the injection device (6) in the tubular guiding device (2) and couples the injection device (6) to the guiding device (1) in the second state of the injection device (6) such that the injection device (6) can be removed from the skin surface (100) after injection of the dosage to the subject together with the guiding device (1) while being coupled to the tubular guiding device (2).

13. The method as claimed in claim 12, further comprising:
resiliently biasing the at least one positive-fit locking member (24) toward the foremost distal position of the injection device (6) in the tubular guiding device (2).

## Patentansprüche

1. Führungsvorrichtung (1) zum Positionieren und Führen einer Injektionsvorrichtung (6) zur intradermalen Injektion einer Dosis an ein Individuum, wobei die Injektionsvorrichtung (6) einen Spritzenkörper (60) mit einem vorbestimmten Profil an seinem distalen Ende und eine Nadel (68) mit einem distalen Nadelende (69) aufweist, die zum Eindringen in eine Hautoberfläche (100) des Individuums zur intradermalen Injektion der Dosis geeignet ist;
wobei die Vorrichtung (1) umfasst:
eine Basis (4) mit einer Kontaktfläche (42) zur Ausrichtung auf der Hautoberfläche (100) des Individuums, und
eine rohrförmige Führungsvorrichtung (2), die so konfiguriert ist, dass sie eine axiale Bewegung der Injektionsvorrichtung (6) in Richtung der Hautoberfläche (100) von einem zurückgezogenen ersten Zustand, in dem das distale Nadelende (69) nicht in die Hautoberfläche (100) eindringt und durch einen Abschnitt (12) der Führungsvorrichtung (1) gegen die Hautoberfläche abgeschirmt ist, in einen zweiten Zustand führt, in dem das distale Nadelende (69) in die Hautoberfläche (100) des Individuums eindringt, wobei die rohrförmige Führungsvorrichtung (2) eine Mittelachse (L) definiert, die relativ zur Kontaktfläche (42) unter einem spitzen Winkel (α) geneigt ist, und eine distale Öffnung (37) aufweist, durch die das distale Nadelende (69) zur intradermalen Injektion geführt wird, wobei
die Kontaktfläche (42) der Basis (4) eben ist und die rohrförmige Führungsvorrichtung (2) relativ zur Basis (4) unter dem spitzen Winkel (α) geneigt ist, wobei
die rohrförmige Führungsvorrichtung (2) einen hülsenförmigen Führungsabschnitt (32, 33) aufweist, der eng an das vorbestimmte Profil am distalen Ende der Injektionsvorrichtung (6) angepasst ist, um eine Verkippung der Injektionsvorrichtung (6) um die Mittelachse (L) an oder nahe der vordersten distalen Position der Injektionsvorrichtung (6) in der rohrförmigen Führungsvorrichtung (2) zu verhindern, und
die rohrförmige Führungsvorrichtung (2) mindestens ein Verriegelungselement (24) umfasst, das an einer Seite proximal zu dem hülsenförmigen Führungsabschnitt (32, 33) vorgesehen ist und zum Verriegeln der Injektionsvorrichtung (6) an der Führungsvorrichtung (1) in der vordersten distalen Position der Injektionsvorrichtung (6) in der rohrförmigen Führungsvorrichtung (2) und zum Koppeln der Injektionsvorrichtung (6) mit der Führungsvorrichtung (1) in dem zweiten Zustand der Injektionsvorrichtung (6) konfiguriert ist,
**dadurch gekennzeichnet, dass**
das mindestens eine Verriegelungselement (24) ein formschlüssiges Verriegelungselement ist, das dazu ausgebildet ist, die Injektionsvorrichtung (6) in der vordersten distalen Position der Injektionsvorrichtung (6) in der rohrförmigen Führungsvorrichtung (2) formschlüssig mit der Führungsvorrichtung (1) zu verriegeln und die Injektionsvorrichtung (6) in dem zweiten Zustand der Injektionsvorrichtung (6) mit der Führungsvorrichtung (1) zu koppeln, so dass die Injektionsvorrichtung (6) nach der Injektion der Dosis in das Individuum zusammen mit der Führungsvorrichtung (1) von der Hautoberfläche (100) entfernt werden kann und dabei gleichzeitig mit der rohrförmigen Führungsvorrichtung (2) gekoppelt bleibt.

2. Führungsvorrichtung (1) nach Anspruch 1, wobei das mindestens eine formschlüssige Verriegelungselement (24) so konfiguriert ist, dass es die Injektionsvorrichtung (6) elastisch in Richtung der vordersten distalen Position der Injektionsvorrichtung (6) in der rohrförmigen Führungsvorrichtung (2) vorspannt.

3. Führungsvorrichtung (1) nach Anspruch 1 oder 2, wobei
der hülsenförmige Führungsabschnitt (32, 33) eine sich verjüngende Führungshülse (32) in der Nähe eines distalen Endes der rohrförmigen Führungsvorrichtung (2) als Anschlagelement aufweist, um die axiale Bewegung zu begrenzen und die vorderste distale Position der Injektionsvorrichtung (6) in der rohrförmigen Führungsvorrichtung (2) durch Reibschluss und/oder Formschluss zu definieren; oder
der hülsenförmige Führungsabschnitt (32, 33) oder die rohrförmige Führungsvorrichtung (2) ein Anschlagelement aufweist, um die axiale Bewegung zu begrenzen und die vorderste distale Position der Injektionsvorrichtung (6) in der rohrförmigen Führungsvorrichtung (2) durch Zusammenwirken mit der Injektionsvorrichtung (6) zu definieren, wobei das Anschlagelement mindestens einen in dem hülsenförmigen Führungsabschnitt (32, 33) vorgesehenen Vorsprung umfasst.

4. Führungsvorrichtung (1) nach einem der vorhergehenden Ansprüche, wobei die rohrförmige Führungsvorrichtung (2) ferner eine proximale rohrförmige Führungsvorrichtung (11) aufweist, die auf einer Seite proximal des hülsenförmigen Führungsabschnitts (32, 33) angeordnet ist und mindestens einen elastischen Verriegelungsfinger (20) aufweist, wobei
jedes formschlüssige Verriegelungselement (24) an einem jeweiligen federnden Verriegelungsfinger (20) vorgesehen ist, und
jedes formschlüssige Verriegelungselement vorzugsweise als Verriegelungsnase (24) oder Verriegelungsvertiefung ausgebildet ist, die einstückig mit dem jeweiligen elastischen Verriegelungsfinger (20) ausgebildet und so konfiguriert ist, dass diese mit einem entsprechenden Vorsprung (65) oder einer entsprechenden Vertiefung zusammenwirkt, der bzw. die an der Injektionsvorrichtung (6) vorgesehen ist.

5. Führungsvorrichtung (1) nach Anspruch 4, wobei die proximale rohrförmige Führungsvorrichtung (11) durch eine untere Seitenwand (12), die sich parallel zur Mittelachse (L) erstreckt, und eine Vielzahl von elastischen Verriegelungsfingern (20) ausgebildet ist, die in gleichwinkligen Abständen um die Mittelachse (L) herum und gegenüber der unteren Seitenwand (12) angeordnet sind, wobei jeder elastische Verriegelungsfinger (20) eine sich verjüngende Führungsfläche (23) aufweist, die in Richtung auf das jeweilige formschlüssige Verriegelungselement (24) verjüngt ist.

6. Führungsvorrichtung (1) nach Anspruch 4 oder 5, wobei jeder elastische Verriegelungsfinger (20) eine sich verjüngende Führungsfläche (23) aufweist, die sich zum distalen Ende der proximalen rohrförmigen Führungsvorrichtung (2) hin verjüngt und in der Verriegelungsnase (24) endet, die von einem jeweiligen Schaft (25) des jeweiligen elastischen Verriegelungsfingers (20) vorsteht.

7. Führungsvorrichtung (1) nach Anspruch 6, wobei der Schaft (25) des jeweiligen elastischen Verriegelungsfingers (20) zusammen mit einer Flanke der jeweiligen Verriegelungsnase (24) und einer radialen Stufe (26), die entlang einer Richtung der Mittelachse (L) gegenüberliegt, eine Aufnahme bildet, die in einer Querschnittsansicht allgemein eine rechteckige Form aufweist.

8. Führungsvorrichtung (1) nach einem der vorhergehenden Ansprüche, wobei die rohrförmige Führungsvorrichtung (2) ferner eine zylindrische Führungshülse (33) umfasst, die an ihrem distalen Ende vorgesehen ist und so konfiguriert ist, dass diese mit einem distalen Ende der Injektionsvorrichtung (6) an oder in der Nähe der vordersten distalen Position der Injektionsvorrichtung (6) in der rohrförmigen Führungsvorrichtung (2) zusammenwirkt, um die Injektionsvorrichtung (6) in der rohrförmigen Führungsvorrichtung (2) zu zentrieren, wenn das distale Nadelende (69) zur intradermalen Injektion in die Hautoberfläche (100) des Individuums eindringt.

9. Führungsvorrichtung (1) nach einem der vorhergehenden Ansprüche, wobei die Injektionsvorrichtung (6) ferner eine rohrförmige Nadelhalterung (66) aufweist, die am distalen Ende des Spritzenkörpers (60) angebracht ist, und die rohrförmige Nadelhalterung (66) als ein Sitz ausgebildet ist, der die Nadel (68) festhält und eine Fluidverbindung zwischen der Nadel (68) und einem Aufbewahrungsvolumen (61) des Spritzenkörpers (60) herstellt.

10. Führungsvorrichtung (1) nach Anspruch 9, wobei die rohrförmige Nadelhalterung (66) einen sich verjüngenden Abschnitt (67) und einen Abschnitt (66c) mit einem zylindrischen Außenprofil aufweist, der an einer Seite distal zu dem sich verjüngenden Abschnitt (67) angeordnet ist.

11. Medizinische Vorrichtung zur intradermalen Injektion einer Dosis an ein Individuum, umfassend
eine Injektionsvorrichtung (6), die einen Spritzenkörper (60) mit einem vorbestimmten Profil an seinem distalen Ende und eine Nadel (68) mit einem distalen Nadelende (69) umfasst, das so angepasst ist, dass es eine Hautoberfläche (100) des Individuums zur intradermalen Injektion der Dosis durchdringt, und
eine Führungsvorrichtung (1) nach einem der vorhergehenden Ansprüche zum Führen der axialen Bewegung der Injektionsvorrichtung (6) in Richtung der Hautoberfläche (100) von einem zurückgezogenen ersten Zustand, in dem das distale Nadelende (69) nicht in die Hautoberfläche (100) eindringt und durch einen Abschnitt (12) der Vorrichtung (1) gegen die Hautoberfläche abgeschirmt ist, in einen zweiten Zustand, in dem das distale Nadelende (69) in die Hautoberfläche (100) des Individuums eindringt.

12. Verfahren zur Kontrolle bzw. Einstellung des Winkels und der Eindringtiefe einer Nadel in eine Hautoberfläche eines Individuums mittels einer Injektionsvorrichtung (6), die einen Spritzenkörper (60) mit einem vorbestimmten Profil an seinem distalen Ende und eine Nadel (68) mit einem distalen Nadelende (69) umfasst, mit den folgenden Schritten:
Bereitstellen einer Führungsvorrichtung (1) nach einem der Ansprüche 1 bis 10;
Platzieren der ebenen Kontaktfläche (42) der Basis (4) auf der Hautoberfläche (100) des Individuums, um die Basis (4) mit der Hautoberfläche (100) des Individuums auszurichten; und
Einführen einer Injektionsvorrichtung (6) in die rohrförmige Führungsvorrichtung (2) und Vorschieben der Injektionsvorrichtung (6) in Richtung der Hautoberfläche (100) entlang einer Mittelachse (L) der rohrförmigen Führungsvorrichtung (2) von einem zurückgezogenen ersten Zustand, in dem das distale Nadelende (69) nicht in die Hautoberfläche (100) eindringt und durch einen Abschnitt (12) der Führungsvorrichtung (1) gegen die Hautoberfläche abgeschirmt ist, in einen zweiten Zustand, in dem das distale Nadelende (69) in die Hautoberfläche (100) des Individuums eindringt und das mindestens eine formschlüssige Verriegelungselement (24) die Injektionsvorrichtung (6) in der vordersten distalen Position der Injektionsvorrichtung (6) in der rohrförmigen Führungsvorrichtung (2) formschlüssig mit der rohrförmigen Führungsvorrichtung (1) verriegelt und die Injektionsvorrichtung (6) in dem zweiten Zustand der Injektionsvorrichtung (6) mit der Führungsvorrichtung (1) koppelt, um eine Eindringtiefe der Nadel (68) in die Hautoberfläche (100) des Individuums zu definieren, zur Kontrolle bzw. Einstellung des Winkels und der Eindringtiefe der Nadel in die Hautoberfläche des Individuums zur Injektion und zum Verhindern einer Verkippung der Injektionsvorrichtung (6) um die Mittelachse (L) an oder nahe der vordersten distalen Position der Injektionsvorrichtung (6) in der rohrförmigen Führungsvorrichtung (2) durch Zusammenwirken eines hülsenförmigen Führungsabschnitts (32, 33) der rohrförmigen Führungsvorrichtung (2) mit dem vorbestimmten Profil am distalen Ende der Injektionsvorrichtung (6), wobei
das mindestens eine formschlüssige Verriegelungselement (24) in der vordersten distalen Position der Injektionsvorrichtung (6) in der rohrförmigen Führungsvorrichtung (2) die Injektionsvorrichtung (6) mit der Führungsvorrichtung (1) verriegelt und im zweiten Zustand der Injektionsvorrichtung (6) die Injektionsvorrichtung (6) mit der Führungsvorrichtung (1) koppelt, so dass die Injektionsvorrichtung (6) nach der Injektion der Dosis an das Individuum zusammen mit der Führungsvorrichtung (1) von der Hautoberfläche (100) entfernt werden kann und dabei gleichzeitig mit der rohrförmigen Führungsvorrichtung (2) gekoppelt bleibt.

13. Verfahren nach Anspruch 12, weiterhin umfassend
elastisches Vorspannen des mindestens einen formschlüssigen Verriegelungselements (24) in Richtung der vordersten distalen Position der Injektionsvorrichtung (6) in der rohrförmigen Führungsvorrichtung (2).

## Revendications

1. Dispositif de guidage (1) pour positionner et guider un dispositif d'injection (6) pour l'injection intradermique d'un dosage à un sujet, ledit dispositif d'injection (6) comprenant un corps de seringue (60) ayant un profil prédéterminé à son extrémité distale et une aiguille (68) ayant une extrémité d'aiguille distale (69) adaptée pour pénétrer une surface cutanée (100) du sujet pour l'injection intradermique du dosage ;
ledit dispositif de guidage (1) comprenant
une base (4) ayant une surface de contact (42) pour l'alignement avec la surface de la peau (100) du sujet, et
un dispositif de guidage tubulaire (2) configuré pour guider un mouvement axial du dispositif d'injection (6) vers la surface de la peau (100) depuis un premier état rétracté où l'extrémité distale de l'aiguille (69) ne pénètre pas la surface de la peau (100) et est protégée contre la surface de la peau par une partie (12) du dispositif de guidage (1) jusqu'à un second état où l'extrémité distale de l'aiguille (69) pénètre la surface de la peau (100) du sujet, ledit dispositif de guidage tubulaire (2) définissant un axe central (L) incliné par rapport à la surface de contact (42) à un angle aigu (α) et comprenant une ouverture distale (37) devant être traversée par l'extrémité distale de l'aiguille (69) pour l'injection intradermique, dans lequel
la surface de contact (42) de la base (4) est plane et le dispositif de guidage tubulaire (2) est incliné par rapport à la base (4) au niveau dudit angle aigu (α), dans lequel
le dispositif de guidage tubulaire (2) comprend une partie de guidage en forme de manchon (32, 33) qui correspond étroitement au profil prédéterminé à l'extrémité distale du dispositif d'injection (6), afin d'empêcher un basculement du dispositif d'injection (6) autour de l'axe central (L) à la position distale la plus avancée du dispositif d'injection (6) dans le dispositif de guidage tubulaire (2) ou à proximité de cette position, et
le dispositif de guidage tubulaire (2) comprend au moins un élément de verrouillage (24) prévu sur un côté proximal de la partie de guidage en forme de manchon (32, 33) et configuré pour verrouiller le dispositif d'injection (6) au dispositif de guidage (1) dans la position distale la plus avancée du dispositif d'injection (6) dans le dispositif de guidage tubulaire (2) et pour coupler le dispositif d'injection (6) au dispositif de guidage (1) dans le second état du dispositif d'injection (6),
**caractérisé par le fait que**
l'au moins un élément de verrouillage (24) est un élément de verrouillage à ajustement positif configuré pour verrouiller le dispositif d'injection (6) au dispositif de guidage (1) dans la position distale la plus avancée du dispositif d'injection (6) dans le dispositif de guidage tubulaire (2) d'une manière à ajustement positif et pour coupler le dispositif d'injection (6) au dispositif de guidage tubulaire (2) dans le deuxième état du dispositif d'injection (6) de sorte que le dispositif d'injection (6) puisse être retiré de la surface de la peau (100) après l'injection de la dose au sujet en même temps que le dispositif de guidage (1) tout en étant couplé au dispositif de guidage tubulaire (2).

2. Le dispositif de guidage (1) selon la revendication 1, dans lequel l'au moins un élément de verrouillage à ajustement positif (24) est configuré pour solliciter de manière élastique le dispositif d'injection (6) vers la position distale la plus avancée du dispositif d'injection (6) dans le dispositif de guidage tubulaire (2).

3. Le dispositif de guidage (1) selon la revendication 1 ou 2, dans lequel
la partie de guidage en forme de manchon (32, 33) comprend un manchon de guidage conique (32) près d'une extrémité distale du dispositif de guidage tubulaire (2) comme élément d'arrêt pour limiter le mouvement axial et définir la position distale la plus avancée du dispositif d'injection (6) dans le dispositif de guidage tubulaire (2) par au moins l'une des deux méthodes suivantes : l'engagement par friction et l'ajustement positif ; ou
la partie de guidage en forme de manchon (32, 33) ou le dispositif de guidage tubulaire (2) comprend un élément d'arrêt pour limiter le mouvement axial et définir la position distale la plus avancée du dispositif d'injection (6) dans le dispositif de guidage tubulaire (2) par coopération avec le dispositif d'injection (6), ledit élément d'arrêt comprenant au moins une protubérance prévue dans la partie de guidage en forme de manchon (32, 33).

4. Le dispositif de guidage (1) selon l'une quelconque des revendications précédentes, dans lequel le dispositif de guidage tubulaire (2) comprend en outre un dispositif de guidage tubulaire proximal (11) disposé sur un côté proximal de la partie de guidage en forme de manchon (32, 33) et comprenant au moins un doigt de verrouillage élastique (20), dans lequel.
chaque élément de verrouillage à fixation positive (24) est fourni à un doigt de verrouillage élastique respectif (20), et
chaque élément de verrouillage à ajustement positif est de préférence constitué d'un nez de verrouillage (24) ou d'une dépression de verrouillage formée intégralement avec le doigt de verrouillage élastique respectif (20) et configurée pour coopérer avec une protubérance (65) ou une dépression correspondante prévue sur le dispositif d'injection (6).

5. Le dispositif de guidage (1) selon la revendication 4, dans lequel le dispositif de guidage tubulaire proximal (11) est formé d'une paroi latérale inférieure (12) s'étendant parallèlement à l'axe central (L) et d'une pluralité de doigts de verrouillage résilients (20) disposés à intervalles équiangulaires autour de l'axe central (L) et à l'opposé de la paroi latérale inférieure (12), chaque doigt de verrouillage résilient (20) comprenant une surface de guidage conique (23), qui est conique vers l'élément de verrouillage à ajustement positif respectif (24).

6. Le dispositif de guidage (1) selon la revendication 4 ou 5, dans lequel chaque doigt de verrouillage élastique (20) comprend une surface de guidage (23) effilée vers l'extrémité distale du dispositif de guidage tubulaire proximal (2) et se terminant par le nez de verrouillage (24) faisant saillie à partir d'une tige respective (25) du doigt de verrouillage élastique respectif (20).

7. Le dispositif de guidage (1) selon la revendication 6, dans lequel la tige (25) du doigt de verrouillage résilient respectif (20) forme, avec un flanc du nez de verrouillage respectif (24) et une marche radiale (26) opposée le long d'une direction de l'axe central (L), un réceptacle qui est généralement de forme rectangulaire, si on le regarde dans une vue en coupe transversale.

8. Le dispositif de guidage (1) selon l'une quelconque des revendications précédentes, dans lequel le dispositif de guidage tubulaire (2) comprend en outre un manchon de guidage cylindrique (33) prévu à une extrémité distale de celui-ci, configuré pour coopérer avec une extrémité distale du dispositif d'injection (6) à la position distale la plus avancée du dispositif d'injection (6) dans le dispositif de guidage tubulaire (2) ou à proximité de celle-ci, afin de centrer le dispositif d'injection (6) dans le dispositif de guidage tubulaire (2) lorsque l'extrémité distale de l'aiguille (69) pénètre dans la surface de la peau (100) du sujet en vue d'une injection intradermique.

9. Le dispositif de guidage (1) selon l'une quelconque des revendications précédentes, dans lequel le dispositif d'injection (6) comprend en outre une monture d'aiguille tubulaire (66) montée sur l'extrémité distale du corps de seringue (60) et la monture d'aiguille tubulaire (66) est formée comme un siège maintenant de manière fixe l'aiguille (68) et établissant une communication fluidique entre l'aiguille (68) et une chambre de stockage (61) du corps de seringue (60).

10. Le dispositif de guidage (1) selon la revendication 9, dans lequel le support d'aiguille tubulaire (66) comprend une partie conique (67) et une partie (66c) présentant un profil extérieur cylindrique disposé sur un côté distal par rapport à la partie conique (67).

11. Le dispositif médical pour l'injection intradermique d'une dose à un sujet, comprenant
un dispositif d'injection (6) comprenant un corps de seringue (60) ayant un profil prédéterminé à son extrémité distale et une aiguille (68) ayant une extrémité distale d'aiguille (69) adaptée pour pénétrer la surface de la peau (100) du sujet pour l'injection intradermique de la dose, et
un dispositif de guidage (1) selon l'une quelconque des revendications précédentes, pour guider le mouvement axial du dispositif d'injection (6) vers la surface de la peau (100) depuis un premier état rétracté où l'extrémité distale de l'aiguille (69) ne pénètre pas la surface de la peau (100) et est protégée contre la surface de la peau par une partie (12) du dispositif de guidage (1) jusqu'à un second état où l'extrémité distale de l'aiguille (69) pénètre la surface de la peau (100) du sujet.

12. Un procédé de contrôle de l'angle et de la profondeur de pénétration d'une aiguille dans la surface de la peau d'un sujet au moyen d'un dispositif d'injection (6) comprenant un corps de seringue (60) ayant un profil prédéterminé à son extrémité distale et une aiguille (68) ayant une extrémité distale d'aiguille (69), comprenant les étapes suivantes :
fournir un dispositif de guidage (1) selon l'une quelconque des revendications 1 à 10 ;
placer la surface de contact plane (42) de la base (4) sur la surface de la peau (100) du sujet, pour aligner la base (4) avec la surface de la peau (100) du sujet ; et
introduire un dispositif d'injection (6) dans le dispositif de guidage tubulaire (2) et pousser le dispositif d'injection (6) vers la surface de la peau (100) le long d'un axe central (L) du dispositif de guidage tubulaire (2) d'un premier état rétracté où l'extrémité distale de l'aiguille (69) ne pénètre pas la surface de la peau (100) et est protégée contre la surface de la peau par une partie (12) du dispositif de guidage (1) à un second état où l'extrémité distale de l'aiguille (69) pénètre la surface de la peau (100) du sujet et où l'au moins un élément de verrouillage à ajustement positif (24) verrouille l'extrémité distale de l'aiguille (69) sur la surface de la peau (100) du sujet, pour aligner la base (4) avec la surface de la peau (100) du sujet ; et (24) verrouille le dispositif d'injection (6) au dispositif de guidage tubulaire (1) de manière positive dans la position distale la plus avancée du dispositif d'injection (6) dans le dispositif de guidage tubulaire (2) et couple le dispositif d'injection (6) au dispositif de guidage (1) dans le second état du dispositif d'injection (6) pour définir une profondeur de pénétration de l'aiguille (68) dans la surface de la peau (100) du sujet, pour contrôler l'angle et la profondeur de pénétration de l'aiguille dans la surface de la peau du sujet pour l'injection et pour empêcher un basculement du dispositif d'injection (6) autour de l'axe central (L) à la position distale la plus avancée du dispositif d'injection (6) dans le dispositif de guidage tubulaire (2) ou à proximité de celle-ci, par la coopération d'une partie de guidage en forme de manchon (32, 33) du dispositif de guidage tubulaire (2) avec le profil prédéterminé à l'extrémité distale du dispositif d'injection (6), dans lequel
l'au moins un élément de verrouillage à ajustement positif (24) verrouille le dispositif d'injection (6) au dispositif de guidage (1) dans la position distale la plus avancée du dispositif d'injection (6) dans le dispositif de guidage tubulaire (2) et couple le dispositif d'injection (6) au dispositif de guidage (1) dans le second état du dispositif d'injection (6) de sorte que le dispositif d'injection (6) puisse être retiré de la surface de la peau (100) après l'injection de la dose au sujet avec le dispositif de guidage (1) tout en étant couplé au dispositif de guidage tubulaire (2).

13. Le procédé selon la revendication 12, comprenant en outre :
la sollicitation élastique de l'au moins un élément de verrouillage à fixation positive (24) vers la position distale la plus avancée du dispositif d'injection (6) dans le dispositif de guidage tubulaire (2).
